# EUROPEAN PATENT APPLICATION

(11) **EP 1 471 053 A2**
(43) Date of publication of application: **27.10.2004**
(21) Application number: 04007925.3
(22) Date of filing: 01.04.2004
(51) Int. Cl.: C07C 269/04, C07C 213/08, C07C 271/20

(54) **Process using a cyclic carbonate reactant and beta-hydroxyurethanes thereby obtained**

(30) Priority: 24.04.2003 EP 03009307
(71) Applicant: UCB S.A., 1070 Bruxelles (BE)
(72) Inventor: Van Holen, Jurgen, 9000 Gent (BE)
(74) Representative: Destryker, Elise Martine

(57) **Abstract**

The present invention relates to a process wherein a cyclic carbonate compound bearing an electrophilic group is reacted with a compound containing a nucleophilic group, especially an amine group. Such reactions permit to obtain urethane groups useful in polymer preparation, such as polyurethanes, or other urethane-containing polymers.

## Description

The present invention relates to a process wherein an electrophilic group containing cyclic carbonate compound is reacted with a compound containing a nucleophilic function. It also relates to the synthesis of the electrophilic group containing cyclic carbonate compound.

The reaction of a cyclic carbonate compound with a compound containing a nucleophilic group, especially an amine compound, is known. First publications on that subject date from late fifties early sixties: see for example US 2,802,022, US 2,935,494 and US 3,072,613.

When an amine compound (1) is reacted with a cyclic carbonate compound containing a 5-membered ring (1,3-dioxolan-2-one ring) (2), the product obtained is a β-hydroxyurethane (3 and/or 4):

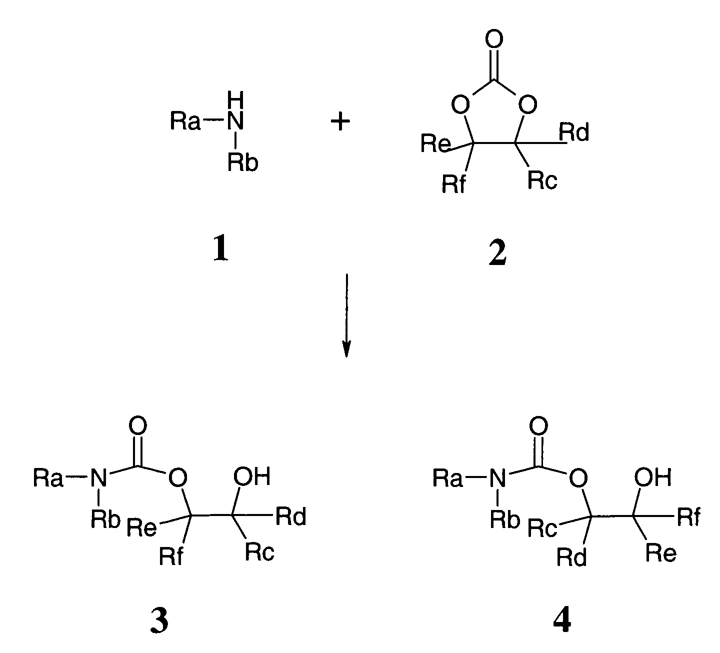

Urethane groups are useful in polymer preparation, such as polyurethanes, or other urethane-containing polymers.

Compared to ordinary urethane groups, betahydroxyurethane groups can provide desirable properties to the formed polymer, namely:
■ the increased resistance towards hydrolysis (Figovski,0., Improving the protective properties of non metallic corrosion resistant materials and coatings. Journal of Medeleev Chemical Society, N.Y., USA 1988 Vol 33 No 3 .pp 31-36)
■ more hydrophilic polymers
■ the extra functional groups make it possible to afterwards crosslink the polymer if desired.

Another advantage of the reaction between amine and cyclic carbonate compounds is that it allows preparation of polyurethane polymers without isocyanate reactants. Such polymers are often called "non-isocyanate" polyurethane polymers. Although the use of di-isocyanates for the synthesis of polyurethanes is widely accepted throughout the industry, there are some disadvantages connected with the NCO chemistry:
■ di-isocyanates are toxic and need special care while handled
■ di-isocyanates with a low vapour pressure can be absorbed easily by the human body (skin, eyes, lungs) and are to be considered as very hazardous chemicals
■ their production involves phosgene while HCl is emitted: this process requires extreme safety measures
■ because of the hydrogenation step and the consequent purification, aliphatic di-isocyanates are expensive and often prevent the use of polyurethanes in a given application for economical reasons.

However, cyclic carbonates react relatively slowly with amines, particularly hindered primary or secondary amines and at ambient (about 25 °C) temperatures. This slow reactivity has limited the usefulness of these types of reactions, for example in coatings which crosslink at ambient temperature. Therefore, means of speeding up such reactions is desired.

A great number of catalysts speeding up such reactions have been already described in literature. For example, US 5,055,542 and US 5,132,458 recommend the use of strongly basic compounds as catalysts for reaction including less reactive secondary amines. Such a strongly basic compound is, for example, diazabicyclooctane (DABCO). So-called supranucleophilic catalysts, for example 4-pyrrolidinopyridine are also suitable here. US 5,977,262 recommends the use of potassium tert-butoxide as catalyst.

However, known catalysts are either not very effective (ammoniumsalts) and/or not suited for an industrial process (very strong bases such as potassium tert-butoxide). Moreover, some reagents (amines) are not compatible with the strong bases. On the other hand, without a catalyst the reaction takes several days.

The present invention provides a process of forming an organic compound, wherein
(a) a component (A) containing at least one cyclic carbonate group having the general formula (I): 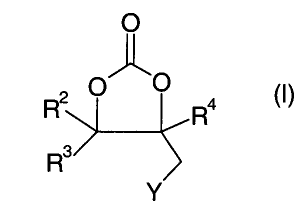 wherein:
   R², R³ and R⁴ are, each independently, chosen from hydrogen, alkyl, alkenyl, wherein alkyl and alkenyl may contain from 0 to 8 ether bridges, and/or may be substituted by one or more aryl, hydroxyl group, and/or cyclic carbonate group of formula (II) 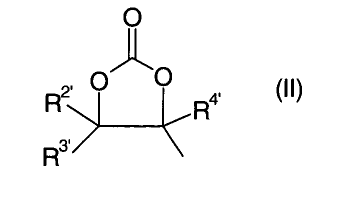 wherein R^{2'}, R^{3'} and R^{4'} are, each independently, chosen from hydrogen, alkyl, alkenyl, wherein alkyl and alkenyl may contain from 0 to 8 ether bridges, and/or may be substituted by one or more aryl, hydroxyl group and/or Y group;
   Y is an electrophilic group selected from ammonium -N⁺(R¹) (R^{1'}) (R^{1"})Z⁻ and phosphonium -P⁺((O)ₙR¹) ((O)ₙR^{1'}) ((O)ₙR^{1''}) Z⁻ , wherein each n, independently, is 0 or 1 and each R¹, R^{1'} and R^{1"} , independently, represents an alkyl optionally substituted by one or more aryl, Y group and/or cyclic carbonate group of formula (III) 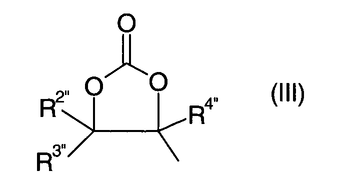 wherein R^{2"}, R^{3"} and R^{4"} are, each independently, chosen from hydrogen, alkyl, alkenyl, wherein alkyl and alkenyl may contain from 0 to 8 ether bridges, and/or may be substituted by one or more aryl and/or hydroxyl group;
   Z- represents an anion;
(b) is reacted with ammonia, hydrazine or an organic compound (B) containing at least one reactive nucleophilic function X wherein each X is, independently, chosen from a primary amino or hydrazo , secondary amino or hydrazo , thiol , hydroxy, and/or oxime;
(c) such that the cyclic carbonate is opened and that an organic compound (C) containing at least one unit of the general formula -X-CO-O- is formed.

It has now surprisingly been found that the presence of at least one electrophilic group containing radical Y, attached to the cyclic carbonate through a methylene bridge, greatly enhance the reaction rate between this cyclic carbonate and a compound [B] containing a reactive nucleophilic function X, such as an amine function for example. The reaction is sufficiently fast, which means that the reaction can be achieved at room temperature.

By organic compound (B) containing a reactive nucleophilic function X which is a primary amino is meant any compound bearing a -NH₂ group attached directly to a carbon atom.

By compound (B) containing a reactive nucleophilic function X which is a secondary amino is meant any compound bearing a -NH group attached directly to 2 carbon atoms.

By compound (B) containing a reactive nucleophilic function X which is a thiol is meant any compound bearing a -SH group attached directly to a carbon atom.

By compound (B) containing a reactive nucleophilic function X which is an hydroxy is meant any compound bearing a -OH group attached directly to a carbon atom.

By compound (B) containing a reactive nucleophilic function X which is an oxime is meant any compound bearing a =N-OH group attached directly to a C atom.

The compound (B) containing a reactive nucleophilic function X which is a primary hydrazo is meant any compound bearing a -NH-NH₂ group directly attached directly to a C atom.

The compound (B) containing a reactive nucleophilic function X which is a secondary hydrazo is meant any compound bearing a -N-NH₂, -NH-NH and/or -N-NH group directly to a C atom.

The term "alkyl", as used herein, is defined as including saturated, monovalent hydrocarbon radicals having straight, branched or cyclic moieties or combinations thereof and containing 1 to 50 carbon atoms.

The term "alkenyl" as used herein, is defined as including straight and cyclic, branched and unbranched, unsaturated hydrocarbon radicals having at least one double bond and containing from 2 to 50 carbon atoms; such as ethenyl (= vinyl), 1-methyl-1-ethenyl, 2-methyl-1-propenyl, 1-propenyl, 2-propenyl (= allyl), 1-butenyl, 2-butenyl, 3-butenyl, 4-pentenyl, 1-methyl-4-pentenyl, 3-methyl-l-pentenyl, 1-hexenyl, 2-hexenyl, and the like. The term "aryl" as used herein, is defined as including an organic radical derived from an aromatic hydrocarbon comprising 1 or more rings by removal of one hydrogen, and containing from 5 to 30 carbon atoms, such as phenyl and naphthyl.

The term "alkylene" as used herein, is defined as including saturated, divalent hydrocarbon radicals having straight, branched or cyclic moieties or combinations thereof and containing 1 to 50 carbon atoms.

The term "alkenylene" as used herein, is defined as including unsaturated, divalent hydrocarbon radicals having straight, branched or cyclic moieties or combinations thereof, containing at least one carbon-carbon double bond and containing 1 to 50 carbon atoms.

The term "arylene" as used herein, is defined as including divalent radicals derived from an aromatic hydrocarbon comprising one or more rings by removal of two hydrogen atoms and containing from 5 to 30 carbon atoms.

The term "aralkylene" as used herein, represents a divalent radical comprising a combination of alkylene and arylene moieties.

By alkyl, alkenyl, alkylene, alkenylene, arylene and aralkylene containing an ether bridge is meant an alkyl, alkenyl, alkylene, alkenylene, arylene or aralkylene radical wherein a carbon atom is replaced by an oxygen atom, forming a group such as -C-O-C-.

By alkyl, alkylene, alkenylene, arylene and aralkylene chain containing tertiary amine bridge is meant such radical wherein a tertiary amine group is present between 2 carbon atoms, forming a group of formula -C-NR-C-, wherein R represents an alkyl or aryl group. In that case, R is preferably an alkyl group containing from 1 to 15 carbon atoms.

Advantages obtainable by the claimed process include :
■ An increase of reaction speed during the synthesis of (poly)urethanes by reacting (poly)amines with (poly)cyclocarbonates
■ Cheap, commercially available (poly)amines can be used for the synthesis of (poly)urethanes. These amines include JEFFAMINE, diethanolamine, piperazine,...
■ Because the reaction speed is increased considerably, even hindered primary and secondary amines can be used in the synthesis.
■ Shorter reaction times and lower curing temperatures also lead to an economical advantage.

The component (A) contains at least one 5-membered cyclic carbonate group. Preferably, the component (A) does not comprise more than 5 cyclic carbonate groups. Compounds comprising 2 to 4 cyclic carbonate groups are particularly preferred, especially in combination with compounds (B) comprising at least 2 reactive functions X when oligomers or polymers are desired.

Components (A) wherein R², R³ and R⁴ are, each independently, chosen from hydrogen and alkyl comprising from 1 to 6 carbon atoms, optionally substituted by one aryl, hydroxyl group, and/or cyclic carbonate group of formula (II) 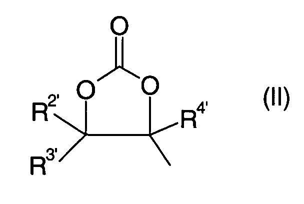 wherein R^{2'}, R^{3'} and R^{4'} are each independently chosen from hydrogen and alkyl comprising from 1 to 6 carbon atoms, optionally substituted by one aryl, hydroxyl group and/or Y group, are preferred. Among these components, those wherein at least two of R², R³ and R⁴ are hydrogen are more preferred.

Components (A) wherein Y is an electrophilic group selected from ammonium - N⁺(R¹) (R^{1'}) (R^{1''})Z⁻ and phosphonium -P⁺((O)ₙR¹) ((O)ₙR^{1'}) ((O)ₙR^{1''}) Z- , wherein at least one of R^{1'}and R^{1"} , independently, are selected from alkyl groups comprising from 1 to 4 carbon atoms, especially methyl, are preferred. Among such components, those wherein R¹ represents an alkyl comprising from 1 to 6 carbon atoms, optionally substituted by aryl and/or cyclic carbonate group of formula (III) 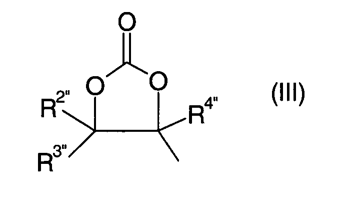 wherein R²", R³" and R⁴" are, each independently, chosen from hydrogen and alkyl comprising from 1 to 6 carbon atoms, optionally substituted by aryl and/or hydroxyl group, are more preferred. Especially preferred are those wherein R¹ is benzyl or a methyl bearing a cyclic carbonate group of formula (III) 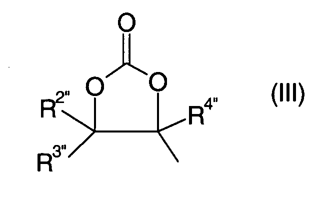 wherein R^{2"}, R^{3"} and R^{4"} are, each independently, chosen from hydrogen and alkyl comprising from 1 to 6 carbon atoms.

Other preferred components (A) are those wherein Y is an electrophilic group selected from ammonium -N⁺(R¹) (R^{1'}) (R^{1"})Z⁻ and phosphonium -P⁺((O)ₙR¹) ((O)ₙR^{1'}) ((O)ₙR^{1"}) Z⁻ , wherein R^{1"} is an alkyl group comprising from 1 to 4 carbon atoms, especially methyl, wherein R¹ represents an alkyl comprising from 1 to 6 carbon atoms substituted by a cyclic carbonate group of formula (III) 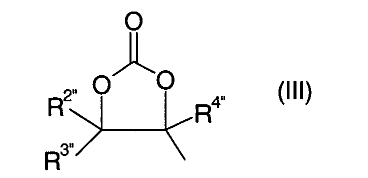 wherein R^{2"}, R^{3"} and R^{4"} are, each independently, chosen from hydrogen and alkyl comprising from 1 to 6 carbon atoms, optionally substituted by aryl and/or hydroxyl group, and wherein R^{1'} is an alkyl substituted by aryl and/or Y group wherein R^{1"} is the same as R^{1"}, and wherein R¹ and R^{1'} are the same as R¹ as defined here above in relation to the ammonium and phosphonium groups. Examples of such components (A) are the tetracarbonates made starting from the tetraglycidylether of metaxylylenediamine derivatives. '

Components (A) wherein Z⁻ is an halide, especially chloride, bromide or iodide are preferred.

Components (A) wherein Y is ammonium -N⁺(R¹) (R^{1'}) (R¹")Z⁻ are most preferred.

Preferred components (A) are chloride, bromide or iodide of 4-(trimethylammoniummethyl)-1,3-dioxolane-2-one, 4-(N-benzyl-N,N-dimethylammoniummethyl)-1,3-dioxolane-2-one and the tetracarbonate made starting from the tetraglycidylether of metaxylylenediamine.

Such 5-membered cyclic carbonate compounds are very desirable because they can be easily prepared starting from cheap epoxides in a reaction with carbondioxide. A reaction of which is already known that it can be catalysed by a lithiumsalt : ref Kihara, N., Hara, N., Endo, T.; J. Org Chem., 1993, 58, 6198-6202.

According to the invention, component (A) may be prepared by reacting compounds (A) where the electrophilic group Y is chloride, bromide or iodide with a nucleophilic compound such as a tertiary (trialkyl)amine, or a trialkyl phosphine or phosphite. A catalyst may eventually be used for accelerating the reaction. These catalysts are selected between quaternary ammonium and phosphonium salts such as tetra-n-butylammonium chloride, tetramethylammonium bromide, benzyltrimethylammonium chloride, tetra-n-butylphosphonium bromide and the like. Alternatively, components (A) may be obtained by reacting corresponding component (A) wherein Y is a tertiary amine or phosphine with an alkylhalide. In a variant of this synthesis, a precursor of such component (A) can be used containing epoxy groups in stead of cyclic carbonate groups, the latter being generated by reaction with CO₂ after the quatemization with the alkylamine or phosphine.

Although component (A) can be reacted with ammonia or hydrazine, it is preferred to react component (A) with an organic component (B) as defined here above.

Preferably, the component (B) contains at least one nucleophilic function X which is an amino group.

Preferably, component (B) is an amine of formula (IX), (X), (XI) or (XII) 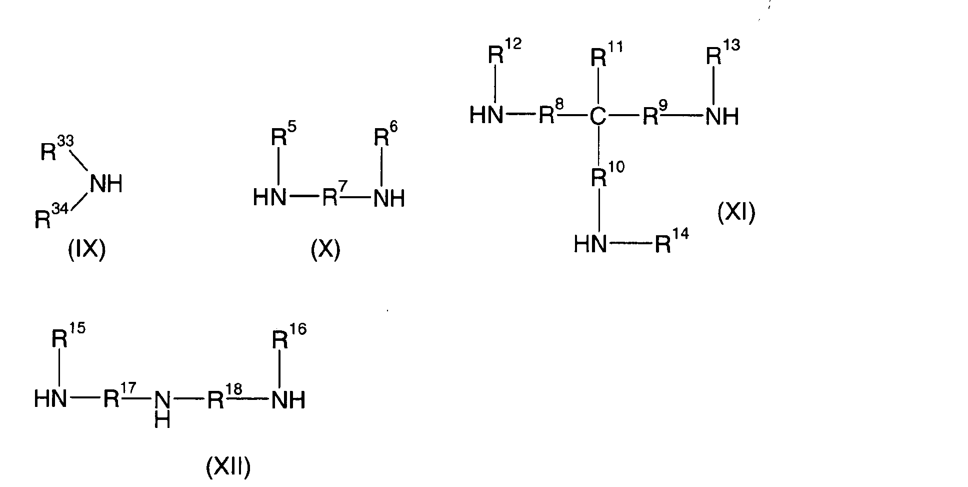 wherein
R³³ is an alkyl, optionally substituted by hydroxy, tertiary amine and/or aryl, and optionally containing from 1 to 20 ether bridges and/or from 1 to 3 tertiary amine bridges,
R³⁴, R⁵, R⁶, R¹², R¹³, R¹⁴, R¹⁵ and R¹⁶ are, independently, chosen from the group of
   ■ hydrogen, and
   ■ alkyl, optionally substituted by hydroxy, tertiary amine and/or aryl, and optionally containing from 1 to 8 ether bridges and/or from 1 to 3 tertiary amine bridges,
   ■ with the proviso that, respectively, R³³ and R³⁴ , R⁵ and R⁶ , R¹² and/or R¹³
and/or R¹⁴, R¹⁵ and R¹⁶ may be linked together in order to form a ring, R⁷, R⁸, R⁹, R¹⁰, R¹⁷ and R¹⁸ are, independently, chosen from alkylene, alkenylene, arylene and aralkylene chains which may contain from 1 to 8 ether bridges and/or from 1 to 3 tertiary amine bridges, R¹¹ is hydrogen or alkyl.

In the amines of formula (IX) R³³ is preferably an alkyl, optionally substituted by hydroxy, tertiary amine and/or aryl, and optionally containing from 1 to 20 ether bridges. Most preferably, R³³ is chosen from the group of alkyl comprising up to 10 carbon atoms, optionally substituted by one hydroxy or tertiary amine and/or optionally containing one or two ether bridges. Non-limiting examples are R³³ substituents chosen from the group of n-propyl, isopropyl, n-butyl, isobutyl, sec-butyl, tert-butyl, n-hexyl, n-octyl, 2-ethylhexyl, isononyl, cyclopentyl, cyclohexyl, 2-methylcyclohexyl, N,N-(di-tert-butyl)ethyl, benzyl, 2-(2-hydroxyethoxy)ethyl, 5-hydroxypentyl, 2-hydroxyethyl, 2-hydroxypropyl, 3-hydroxypropyl, 3-(diethylamino)propyl, 2-(diethylamino)ethyl, 1-methyl-4-(diethylamino)butyl, 2-((di-tert-butyl)amino)ethyl, 3-(dimethylamino)propyl, 2-methoxyethyl, 2-ethoxyethyl, 3-methoxypropyl, 2-methoxyisopropyl, 3-ethoxypropyl, 3-isopropoxypropyl, 3-(2-methoxyethoxy)propyl, 3-(2-ethylhexyloxy)propyl, CH₃O(CH₂CH₂O)₆-(CH₂CHR-O)₁₀-CH₂-CH(CH₃)- wherein R is H or CH₃ in a proportion of 1:9, ethyl, methyl, 1,2-dimethylpropyl.

In the amines of formula (IX) R³⁴ is preferably chosen from the group of hydrogen and alkyl, optionally substituted by hydroxy, tertiary amine or aryl, and optionally containing from 1 to 8 ether bridges. Most preferably, R³⁴ is chosen from the group of hydrogen and alkyl comprising up to 10 carbon atoms, optionally substituted by one hydroxy or tertiary amine and/or optionally containing one or two ether bridges. Non-limiting examples are R³⁴ substituents chosen from the group of hydrogen, ethyl, n-propyl, isopropyl, n-hexyl, methyl, tert-butyl, n-butyl, isobutyl, n-octyl, 2-ethylhexyl, 1,2-dimethylpropyl, cyclohexyl, 2-hydroxyethyl, 2-hydroxyisopropyl, 3-hydroxypropyl, 2-methoxyethyl, 3-(dimethylamino)propyl.

In the amines of formula (X), (XI) and (XII) R5, R⁶, R¹², R¹³, R¹⁴, R¹⁵ and R¹⁶ are preferably, independently, chosen from the group of hydrogen and alkyl comprising up to 10 carbon atoms, most preferably up to 6 carbon atoms.

In the amines of formula (IX), (X), (XI) and (XII) R³³ and R³⁴, R⁵ and R⁶, R¹² and/or R¹³ and/or R¹⁴, R15 and R¹⁶, respectively, may be linked together in order to form a ring. In this case, these substituents are preferably linked so that they form an alkylene chain comprising from 2 to 7 carbon atoms, and optionally containing 1 or 2 ether bridges. In case of R³³ and R³⁴, this alkylene chain is preferably such that a 5 to 7-membered ring is formed, for example a pyrolidine ring, a piperidine ring or a morpholine ring, which may further be substituted by alkyl groups. In case of R6' and R⁶, this alkylene chain is preferably such that a 5 to 7-membered ring is formed, for example piperazine, which may further be substituted by alkyl groups.

In the amines of formula (X) R⁷ is preferably chosen from the group of alkylene and aralkylene chains, containing up to 20 carbon atoms and which may contain from 1 to 8 ether bridges and/or from 1 to 3 tertiary amine bridges. Most preferably, R⁷ is chosen from the group of ethylene, 1,2-propylene, trimethylene, hexamethylene, 2,2-dimethylpropylene, 1-methyltrimethylene, 1,2,3-trimethyltetramethylene, 2-methyl-pentamethylene, 2,2,4-(or 2,4,4-)trimethylhexamethylene, metaxylylene, 3,5,5-trimethylcyclohexyl-1-ene-3-methylene, bis(cyclohexyl-4-ene)methane, bis(4-methylcyclohexyl-3-ene)methane, cyclohexyl-1,3-ene, cyclohexyl-1,4-ene, 1,4-bis(propoxyl-3-ene)butane, N,N-bis(trimethylene)methylamine, 3,6-dioxaoctylene, 3,8-dioxadodecylene, 4,7,10-trioxatridecylene, poly(oxytetramethylene), poly(oxypropylene) with 2 to 15 1,2-propylene oxide units, poly(oxypropylene-co-oxyethylene) with 2 to 15 propylene oxide and 2 to 15 ethylene oxide units, 2,2-dimethylpropylene.

In the amines of formula (XI) R⁸, R⁹, R¹⁰ are preferably, independently, chosen from the group of alkylene, optionally containing from 1 to 8 ether bridges. Most preferably R⁸, R⁹, R¹⁰ are chosen from alkylene comprising up to 15 carbon atoms and containing up to 5 ether bridges.

In the amines of formula (XII) R¹⁷ and R¹⁸ are preferably, independently, chosen from the group of alkylene. Most preferably R¹⁷and R¹⁸ are chosen from alkylene comprising up to 6 carbon atoms.

In the amines of formula (XI) R¹¹ is preferably hydrogen or an alkyl containing from 1 to 4 carbon atoms.

Amines of formula (IX), (X), (XI) and (XII) are known in the art. Amines of formula (IX) which are particularly useful in the process according to the invention are n-propylamine, isopropylamine, n-butylamine, isobutylamine, sec-butylamine, tert-butylamine, 3-methylbutylamine, n-hexylamine, n-octylamine, 2-ethylhexylamine, isononylamine, cyclopentylamine, cyclohexylamine, 2-methylcyclohexylamine, N,N-(di-tert-butyl)ethyleneamine, benzylamine, 2-(2-aminoethoxy)ethanol, 5-aminopentanol, ethanolamine, 1-aminopropan-2-ol, 3-amino-1-propanol, 3-(diethylamino)propylamine, 2-(diethylamino)ethylamine, 1-methyl-4-(diethylamino)butylamine, 2,2-(di-tert-butylamino)ethylamine, 3-(dimethylamino)propylamine, 2-methoxyethylamine, 2-ethoxyethylamine, 3-methoxypropylamine, 1-methoxyisopropylamine, 3-ethoxypropylamine, 3-isopropoxypropylamine, 3-(2-methoxyethoxy)propylamine, 3-(2-ethylhexyloxy)propylamine, polyoxyalkylene amines such as α-oxymethyl-ω-(2-propylamino)-poly(oxypropylene-co-oxyethylene) with an average number of 1,2-propylene oxide units of 9 and an average number of ethylene oxide units of 7, also known as Jeffamine® M-600 (manufactured by Hunstman), diethylamine, di-n-propylamine, diisopropylamine, di-n-hexylamine, N-methylbutylamine, N-ethylbutylamine, di-n-butylamine, diisobutylamine, di-n-octylamine, bis(2-ethylhexyl)amine, N-ethyl-1,2-dimethylpropylamine, dicyclohexylamine, cyclohexylmethylamine, cyclohexylethylamine, N-methylbenzylamine, 2-methylaminoethanol, 2-ethylaminoethanol, 2-butylaminoethanol, diethanolamine, diisopropanolamine, 3-(2-hydroxyethyl)aminopropanol, bis(2-methoxyethyl)amine, bis(3-dimethylaminopropyl)amine, pyrolidine, piperidine, morpholine, 2,6-dimethylmorpholine.

Amines of formula (X) which are particularly useful in the process according to the invention are ethylenediamine, 1,2-propylenediamine, trimethylenediamine, hexamethylenediamine, 2,2-dimethylpropane-1,3-diamine, 1-methyl-1,3-propanediamine, 1,2,3-trimethyl-1,4-butanediamine, 2-methyl-1,5 diaminopentane, 2,2,4-(or 2,4,4-)trimethylhexamethylene diamine, metaxylylenediamine, 1-amino-3-aminomethyl-3,5,5 trimethylcyclohexane (isophorone diamine), bis-(4-aminocyclohexyl)-methane, bis-(4-amino-3-methyl-cyclohexyl)-methane, 1,3-cyclohexanediamine, 1,4-cyclohexanediamine, 1,4-Bis(3-aminopropoxy)butane diamine, N,N-bis(3-aminopropyl)methylamine, triethyleneglycol diamine, 3,3'-(butane-1,4-diylbis(oxy))bispropaneamine, 4,7,10-trioxatridecan-1,13-diamine, polyoxyalkylene diamines such as α-amino-ω-(4-butylamino)-poly(oxytetramethylene), α-amino-ω-(2-propylamino)-poly(oxypropylene) with an average number of 1,2-propylene oxide units of 2.6, also known as Jeffamine® D-230 (manufactured by Hunstman), α-amino-ω-(2-propylamino)- poly(oxypropylene) with an average number of 1,2-propylene oxide units of 5.6, also known as Jeffamine® D-400 (manufactured by Hunstman), α-amino-ω-(2-propylamino)-poly(oxypropylene-co-oxyethylene) with an average number of 1,2 propylene oxide units of 2.5 and ethylene oxide units of 8.5, also known as Jeffamine® ED-600 (manufactured by Hunstman), 2,2-dimethyl-1,3-propanediamine, N,N'-di-tert-butyl-ethanediamine, N,N'-dimethylhexyl-1,6-diamine, piperazine, 1,6-diaminotrimethylhexane, N,N'-dimethyl-1,3-propanediamine and 2,5-dimethylpiperazine.

Amines of formula (XI) that are particularly useful in the process according to the invention is propoxylated trimethylopropane triamine with an average number of number of 1,2-propylene oxide units of 5.3, also known as Jeffamine® T-403 (manufactured by Hunstman).

Amines of formula (XII) that are particularly useful in the process according to the invention are diethylenetriamine, N,N-dimethyldipropylenetriamine, bis(hexamethylene)triamine.

In an embodiment of the invention, compound (B) contains only one primary or secondary amino group.

In other preferred embodiments of the invention, component (B) is a compound containing at least two primary or secondary amino groups. This permits to obtain dimers, oligomers and even polymers when reacted with a component (A) containing at least two linked carbonate rings.

Especially preferred are amines chosen amongst cyclohexylamine, N-methylbutylamine, N-methylbenzylamine, piperidine, piperazine, morpholine, benzylamine, diethylenetriamine, ethanolamine, diethanolamine and polyoxyalkylene amines and diamines.

The organic compound (B) can also be chosen among hydrazo compounds such as mono, di and tri-substituted hydrazines H₂N-NHR²⁹, H₂N-NR²9R³⁰, R²⁹HN-NHR³⁰, R²⁹HN-NR³⁰R³⁵ wherein R²⁹, R³⁰ and R³⁵ are as defined for R³³.

The organic compound (B) can also be chosen among hydroxy compounds such as (polyfunctional) alcohols, for example methanol, ethanol, propanol, isopropanol, butanol, phenol, butanediol, hexanediol, trimethylolpropane, pentaerythritol, glucose, (poly)hydroxy functional oligomers or polymers such as polyesters, polyethers, polycarbonates, polyurethanes, polybutadiene, acrylic polyols, polysiloxanes, starch and the like.

It was found that the reaction temperature between compounds (A) and (B) is not critical and can be comprised between 0 and 120°C. The amount of component (A) and component (B) in the process according to the invention is preferably such that the molar ratio of cyclic carbonate groups to nucleophilic groups X is from 0.5 to 2. In case that a component (A) comprising only one cyclic carbonate group and/or a component (B) comprising only one nucleophilic group X is used, the ratio of amount of such components is preferably such that an almost equivalent ratio of cyclic carbonate groups to nucleophilic groups X is obtained.

The reaction can be made with or without solvent. When a solvent is used, it is preferably chosen among alcohol, ether, ester, dimethylformamide, dimethylsulfoxide, N-methylpyrolidone and water.

The present invention also relates to the products obtainable by the process described here above. More specifically the present invention relates to products comprising at least one -X-CO-O- group and a hydroxy group in β-position of this group and further containing at least one Y-group according to at least one of the structures 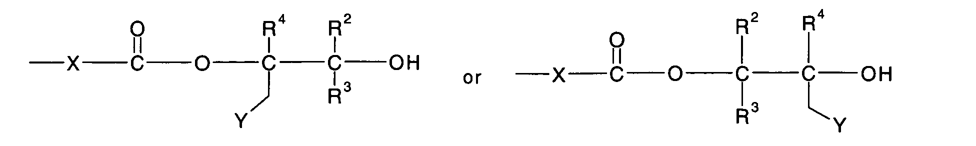 wherein X, R², R³, R⁴ and Y are such as defined here above or, in case R², R³, R⁴ and Y contain a cyclic carbonate group themselves are the structures resulting from the ring-opening of this cyclic carbonate group.

More specifically the present invention relates to β-hydroxyurethanes (products comprising at least one -N-CO-O- group and a hydroxy group in β-position of this urethane group) and further containing at least one Y-group according to at least one of the structures 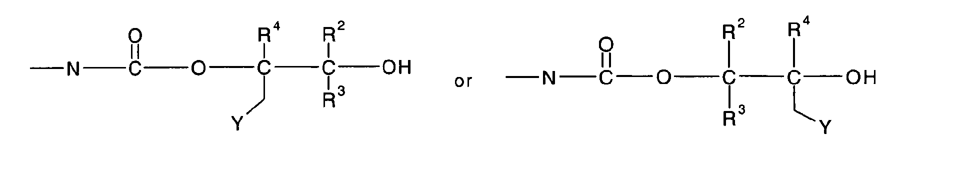 wherein R², R³, R⁴ and Y are such as defined here above or, in case R², R³, R⁴ and Y contain a cyclic carbonate group themselves are the structures resulting from the ring-opening of this cyclic carbonate group.

The invention also extends to a compositions containing a nonisocyanate polyurethane polymer or oligomer comprising urethane functions -NCOO- which contain ternary-substituted nitrogen and bear an hydroxy group in beta-position (β-hydroxyurethane), especially those obtained from the reaction of an amine of formula (X) and a cyclic carbonate component (A) wherein Y is an ammonium.

The invention more specifically relates to compounds responding to one of the following formula or their mixtures

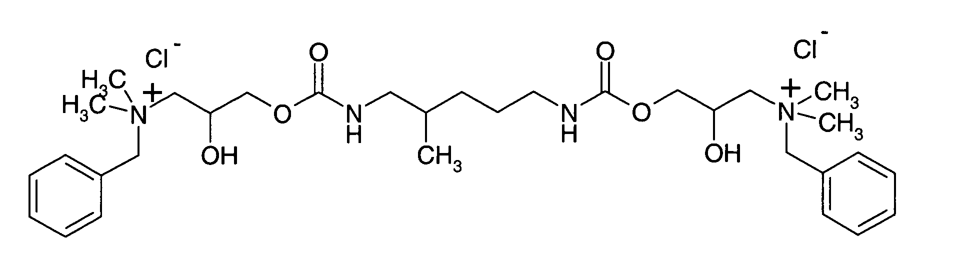

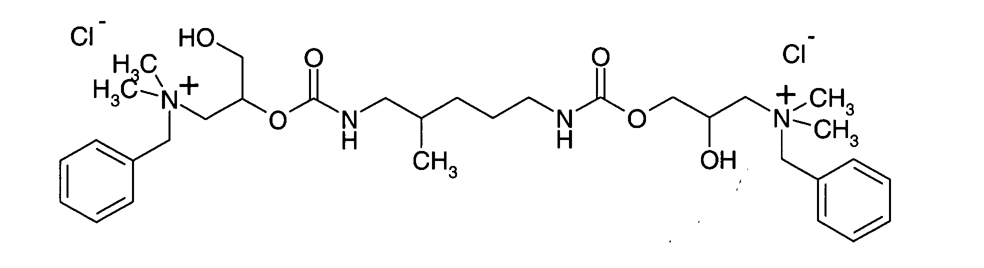

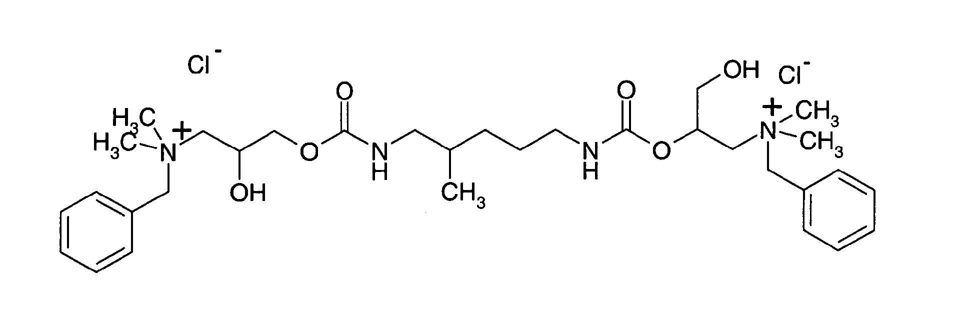

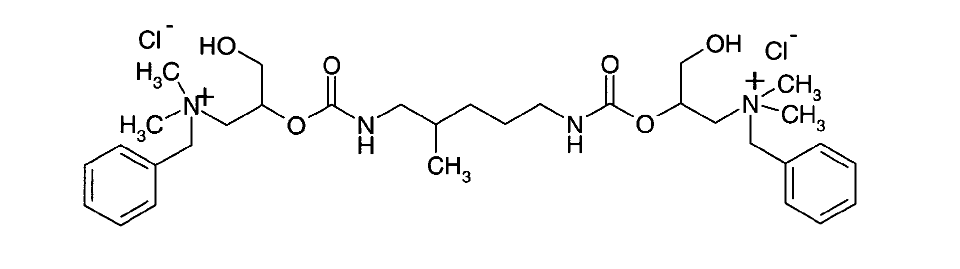

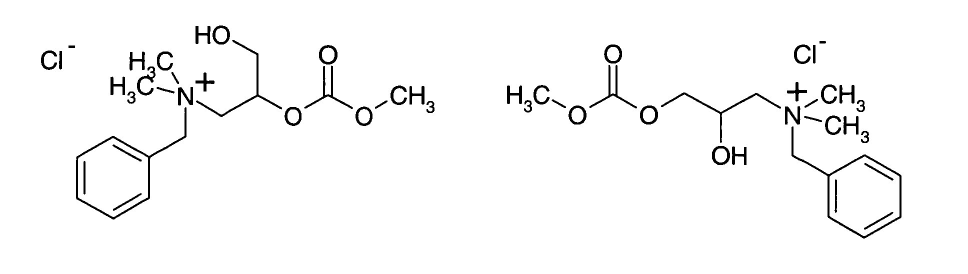

The invention will now be illustrated by examples.

### Example 1 :

### Reaction of N-benzyldimethylamine with 4-chloromethyl-1,3-dioxolan-2-one

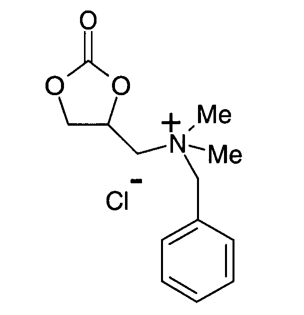

In a 200 ml flask were added N-benzyldimethylamine (60 gram), 4-chloromethyl-1,3-dioxolan-2-one (50 gram) and catalyst tetrabutylammoniumiodide. (2 gram) The mixture is heated to 100°C. The reaction is followed by potentiometrically titrating the remaining amine. When the reaction is finished the precipitate is filtered, washed with diethylether and dried, affording 20 grams of product. The final product has a carbonate content of 3.6 mmol carbonate/gram (theory 3.7 mmol/gr) and a chlorine content of 12 % (theory 13%)

### Example 2 and comparative example :

Example 2 and comparative example were run by the same general procedure. For example 2 : 0.136g (0.0005 mole) of the ammoniumcarbonate prepared in example 1 was dissolved into 4 ml DMSO d6. Then 0.0300g (0.00026 M) 2-methyl-1,5-pentanediamine is added and mixed together, just prior to taking the NMR spectrum. 600µL of this solution was transferred to the NMR tube. For the comparative example the same procedure was used except that instead of the carbonate of example 1 0.044g (0.0005 mole) of ethylenecarbonate was used The NMR-spectra were taken on a "Bruker" 300 MHz spectrometer The integrated NMR-values observed and used for calculation the resulting urethane are located at δ = 7.55 ppm and δ = 5.6 ppm for example 2, resp. the phenyl group of the carbonate of this invention and the CH2-O-CO-NH group of the resulting urethane, and δ = 4.95 ppm and δ = 4.5 ppm for the comparative example, resp. the (CH2)2 signal of ethylenecarbonate and the CH2O-CO-NH group of the resulting urethane.

Table 1 shows the decrease in cyclic carbonate concentration for example 2. For comparison, the same reaction was run but the carbonate of this invention was replaced with ethylenecarbonate. The time given is taken from the time of initial mixing of the components.

**Table 1**

| Example 2 Carbonate of this invention | | Comparative example Ethylenecarbonate | |
|---|---|---|---|
| Time (min) | Relative fraction of remaining carbonate | Time (min) | Relative fraction of remaining carbonate |
| 6 | 90 | 12 | 98.9 |
| 60 | 65 | 600 | 97.4 |
| 960 | 30 | 1200 | 94.3 |
| 1560 | 25 | 1800 | 91.3 |
| 2460 | 19 | 2670 | 86.4 |

### Example 3

0.05 gram of the carbonate of example 1 is added to 1 ml of methanol. The reaction is monitored with NMR. The remaining fraction of carbonate is listed in table 2. As the characteristic signal of the carbonate of example 1 at δ = 5.6 ppm is disappearing a new singlet at δ = 3.1 ppm appears. The cyclic carbonate reacts with the methanol and forms a linear carbonate as is evidenced by the NMR.

**Table 2**

| Example 3 Carbonate of this invention | |
|---|---|
| Time (min) | Relative fraction of remaining carbonate |
| 0 | 100 |
| 60 | 79 |
| 120 | 60 |

### Example 4 :

### Example 4 was run by the same general procedure as example 2.

0.136g (0.0005 mole) of the ammoniumcarbonate prepared in example 1 was dissolved into 4 ml deuterated methanol. Then 0.0300g (0.00026 mole) 2-methyl-1,5-pentanediamine is added and mixed together, just prior to taking the NMR spectrum. 600µL of this solution was transferred to the NMR tube. The NMR-spectra were taken on a "Bruker" 300 MHz spectrometer After 5 minutes the NMR spectrum shows that the characteristic signal for the ammonium carbonate has almost completely disappeared, indicating an extremely fast reaction.

## Claims

1. Process of forming an organic compound wherein
(a) a component (A) containing at least one cyclic carbonate group having the general formula (I): 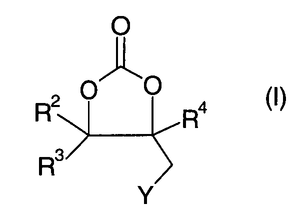 wherein:
R², R³ and R⁴ are, each independently, chosen from hydrogen, alkyl, alkenyl, wherein alkyl and alkenyl may contain from 0 to 8 ether bridges, and/or may be substituted by one or more aryl, hydroxyl group, and/or cyclic carbonate group of formula (II) 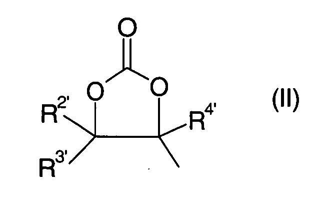 wherein R^{2'}, R^{3'} and R^{4'} are, each independently, chosen from hydrogen, alkyl, alkenyl, wherein alkyl and alkenyl may contain from 0 to 8 ether bridges, and/or may be substituted by one or more aryl, hydroxyl group and/or Y group;
Y is an electrophilic group selected from ammonium -N⁺(R¹ ) (R^{1'} ) (R^{1"})Z⁻ and phosphonium-P⁺((O)ₙR¹) ((O)ₙR^{1'}) ((O)ₙR^{1"}) Z⁻ , wherein each n, independently, is 0 or 1 and each R¹, R^{1'} and R^{1"} , independently, represents an alkyl optionally substituted by one or more aryl, Y group and/or cyclic carbonate group of formula (III) 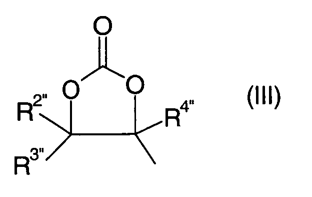 wherein R^{2"}, R^{3"} and R^{4"} are, each independently, chosen from hydrogen, alkyl, alkenyl, wherein alkyl and alkenyl may contain from 0 to 8 ether bridges, and/or may be substituted by one or more aryl and/or hydroxyl group;
Z- represents an anion;
(b) is reacted with ammonia, hydrazine or an organic compound (B) containing at least one reactive nucleophilic function X wherein each X is, independently, chosen from a primary amino or hydrazo , secondary amino or hydrazo , thiol , hydroxy, and/or oxime;
(c) such that the cyclic carbonate is opened and that an organic compound (C) containing at least one unit of the general formula -X-CO-O- is formed.

2. Process according to claim 1, wherein component (A) contains at least two carbonate cycles.

3. Process according to any of claims 1 or 2, wherein component (A) is chosen from 4-(trimethylammoniummethyl)-1,3-dioxolane-2-one, 4-(N-benzyl-N,N-dimethylammoniummethyl)-1,3-dioxolane-2-one and the tetracarbonate made starting from the tetraglycidylether of metaxylylenediamine.

4. Process according to any preceding claim, wherein an organic compound (B) which contains at least one nucleophilic function X which is an amino group is used.

5. Process according to claim 4, wherein component (B) is an amine of formula (IX), (X), (XI) or (XII) 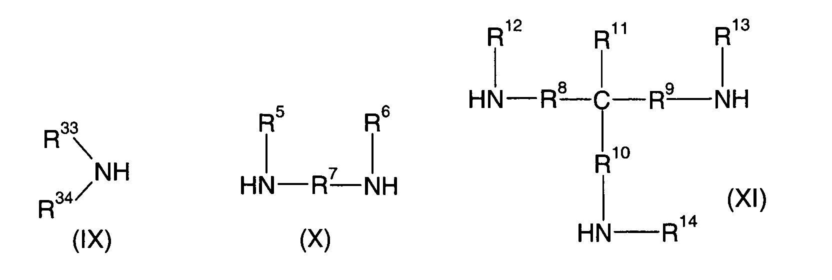 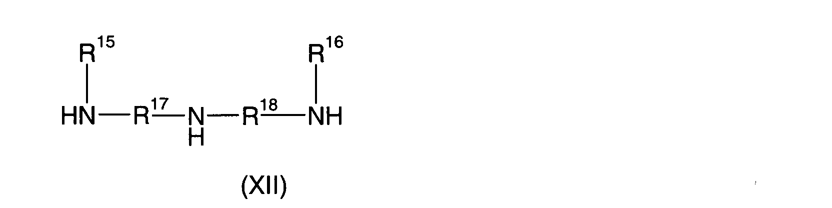 wherein
R³³ is an alkyl, optionally substituted by hydroxy, tertiary amine and/or aryl, and optionally containing from 1 to 20 ether bridges and/or from 1 to 3 tertiary amine bridges,
R³⁴, R⁵, R⁶, R¹², R¹³, R¹⁴, R15 and R¹⁶ are, independently, chosen from the group of
■ hydrogen, and
■ alkyl, optionally substituted by hydroxy, tertiary amine and/or aryl, and optionally containing from 1 to 8 ether bridges and/or from 1 to 3 tertiary amine bridges,
■ with the proviso that, respectively, R³³ and R³⁴ , R⁵ and R⁶ , R¹² and/or R¹³ and/or R¹⁴, R¹⁵ and R¹⁶ may be linked together in order to form a ring,
R⁷, R⁸, R⁹, R¹⁰, R¹⁷ and R¹⁸ are, independently, chosen from alkylene, alkenylene, arylene and aralkylene chains which may contain from 1 to 8 ether bridges and/or from 1 to 3 tertiary amine bridges, R¹¹ is hydrogen or alkyl.

6. Process according to any of claims 4 or 5, wherein component (B) contains at least two primary or secondary amino groups.

7. Process according to any of claims 4 to 6, wherein compound (B) is an amine chosen amongst cyclohexylamine, N-methylbutylamine, N-methylbenzylamine, piperidine, piperazine, morpholine, benzylamine, diethylenetriamine, ethanolamine, diethanolamine and polyoxyalkylene amines and diamines.

8. Process according to any preceding claim, wherein the reaction temperature is comprised between 0 and 120°C.

9. Process according to any preceding claim, wherein the amount of component (A) and compound (B) are such that the molar ratio of cyclic carbonate groups to nucleophilic groups X is from 0.5 to 2.

10. Process according to any preceding claim, wherein the reaction is made in a solvent chosen among: alcohol, ether, ester, dimethylformamide, dimethylsulfoxide, N-methylpyrolidone and water.

11. Process according to any preceding claim, wherein component (A) is prepared by reacting compounds (A) where the electrophilic group Y is chloride or bromide or iodide with a nucleophilic compound such as a tertiary (trialkyl)amine, or a trialkyl phosphine or phosphite.

12. Products obtainable by the process according to any of claims 1 to 11 comprising at least one -X-CO-O- group and a hydroxy group in β-position of said -X-CO-O-group and at least one Y-group according to at least one of the structures 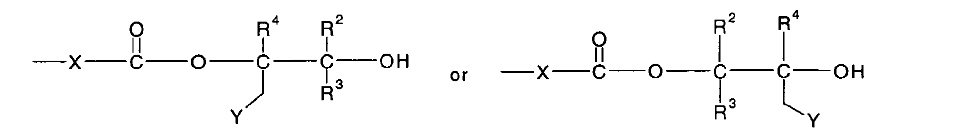 wherein X, R², R³, R⁴ and Y are such as defined in claim 1 or, in case R², R³, R⁴ and Y contain a cyclic carbonate group themselves, the structures resulting from the ring-opening of said cyclic carbonate group.

13. Products according to claim 12 wherein X is N.

14. Products according to claim 13 responding to one of the following formula or their mixtures
